# EUROPEAN PATENT APPLICATION

(11) **EP 4 356 900 A1**
(43) Date of publication of application: **24.04.2024**
(21) Application number: 22811660.4
(22) Date of filing: 26.05.2022
(51) Int. Cl.: A61K 9/00, A61K 47/40, A61K 47/02, A61K 47/26, A61K 31/4015, A61P 1/04

(54) **NOVEL INJECTABLE FORMULATION COMPRISING 1-(5-(2,4-DIFLUOROPHENYL)-1-((3-FLUOROPHENYL)SULFONYL)-4-METHOXY-1H-PYRROL-3-YL)-N-METHYLMETHANAMINE**

(30) Priority: 26.05.2021 KR 20210067636
(71) Applicant: Daewoong Pharmaceutical Co., Ltd., Hwaseong-si, Gyenoggi-do 18623 (KR)
(72) Inventor: JUNG, Yeon Jin, Gunpo-si Gyeonggi-do 15884 (KR); HONG, Eun Ji, Seongnam-si Gyeonggi-do 13245 (KR); KIM, Gyoung Won, Yongin-si Gyeonggi-do 17000 (KR); CHO, Sang Eun, Yongin-si Gyeonggi-do 16909 (KR); KIM, Gwan Young, Yongin-si Gyeonggi-do 13568 (KR)
(74) Representative: Germain Maureau
(86) International application number: PCT/KR2022/007479
(87) International publication number: WO 2022/250469

(57) **Abstract**

The present disclosure relates to a formulation for injection comprising 1-(5-(2,4-difluorophenyl)-1-((3-fluorophenyl)sulfonyl)-4-methoxy-1 H-pyrrol-3-yl)-N-methylmethanamine.

## Description

### [TECHNICAL FIELD]

### CROSS-REFERENCE TO RELATED APPLICATION(S)

This application claims the benefit of Korean Patent Application No. 10-2021-0067636 filed on May 26, 2021 and Korean Patent Application No. 10-2022-0064451 filed on May 26, 2022 in the Korean Intellectual Property Office, the disclosures of which are incorporated herein by reference in their entirety.

The present disclosure relates to a new formulation for injection comprising 1-(5-(2,4-difluorophenyl)-1-((3-fluorophenyl)sulfonyl)-4-methoxy-1H-pyrrol-3-yl)-N-methylmethanamine.

### [BACKGROUND ART]

It is known that even in the case of a formulation containing the same active ingredient, pharmaceutically important properties such as solubility, dissolution characteristics and bioavailability of the active ingredient may differ depending on additional components contained in the formulation. Therefore, along with the development of new compounds, it is also very important to develop a component contained in the formulation that can maximize the pharmacological effect of the developed compounds.

Meanwhile, 1-(5-(2,4-difluorophenyl)-1-(-fluorophenyl)sulfonyl)-4-methoxy-1H-pyrrol-3-yl)-N-methylmethanamine is a substance described in Korean Patent Registration No. 10-1613245, which is a substance that has excellent anti-ulcer activity (i.e., proton pump inhibitory activity, etc.), anti-Helicobacter pylori (H. pylori) activity and GPCR inhibitory activity, and thus is useful for the prevention and treatment of gastrointestinal ulcers, gastritis, reflux esophagitis, or gastrointestinal damage caused by Helicobacter pylori.

However, the above substance or its hydrochloride shows a problem that the stability is poor, for example, the water solubility is low in a neutral pH environment, and acid decomposition products are increased in an acidic environment with good solubility (2.17 mg/ml, pH 6.8), whereby there was a great difficulty in formulating for injection through dissolution and stabilization in an aqueous solution. Therefore, in order to develop a formulation for injection that can achieve a balance between solubility and stability, there is a growing need to study the adjustment of the appropriate pH and the combination of ingredients other than active pharmaceutical ingredients.

Therefore, the present inventors have attempted to formulate for injection by improving the solubility and stability of 1-(5-(2,4-difluorophenyl)-1-((3-fluorophenyl)sulfonyl)-4-methoxy-1H-pyrrol-3-yl)-N-methylmethanamine, and as a result, confirmed that when a specific isotonizing agent is contained within a specific pH range, the above problems can be solved, thereby completing the present disclosure.

### [DETAILED DESCRIPTION OF THE INVENTION]

### [Technical Problem]

It is an object of the present disclosure to provide a new formulation for injection comprising 1-(5-(2,4-difluorophenyl)-1-((3-fluorophenyl)sulfonyl)-4-methoxy-1H-pyrrol-3-yl)-N-methylmethanamine or a pharmaceutically acceptable salt thereof, having high solubility and excellent stability.

### [Technical Solution]

According to an embodiment of the present disclosure, there is provided a formulation for injection comprising: a compound represented by the following Chemical Formula 1 or a pharmaceutically acceptable salt thereof; a cyclodextrin; and an isotonizing agent, wherein the formulation for injection has a pH of 4.0 to 6.0:

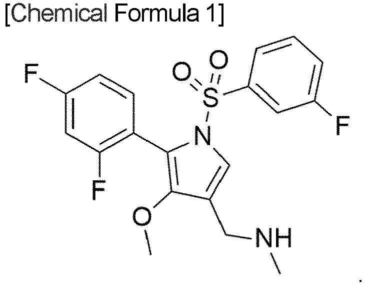

The chemical name of the compound represented by Chemical Formula 1 is 1-(5-(2,4-difluorophenyl)-1-((3-fluorophenyl)sulfonyl)-4-methoxy-1H-pyrrol-3-yl)-N-methylmethanamine, which is a substance described in Korean Patent Registration No. 10-1613245.

The compound represented by Chemical Formula 1 is an active ingredient exhibiting the pharmacological effect of the formulation for injection of the present disclosure, which is a substance that has excellent anti-ulcer activity (i.e., proton pump inhibitory activity, etc.), anti-Helicobacter pylori (H. pylori) activity and GPCR inhibitory action and thus is useful for the prevention and treatment of gastrointestinal ulcers, gastritis, reflux esophagitis, or gastrointestinal damage caused by Helicobacter pylori.

In addition, as an active ingredient exhibiting the pharmacological effect of the formulation for injection of the present disclosure, not only the compound represented by Chemical Formula 1 but also a pharmaceutically acceptable salt thereof can be used. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to ae patient and activates effectively and whose side effects do not degrade the beneficial efficacy of the above compound.

The compound represented by Chemical Formula 1 or a pharmaceutically acceptable salt thereof can obtain pharmaceutically acceptable salts by conventional methods using inorganic or organic acids. For example, the pharmaceutically acceptable salt can be prepared by dissolving the compound represented by Chemical Formula 1 in a water-miscible organic solvent, e.g., acetone, methanol, ethanol or acetonitrile, followed by adding an organic acid or an inorganic acid, and filtering and drying the precipitated crystals. Alternatively, it can be prepared by subjecting a solvent or an excessive amount of acid from the acid-added reaction mixture to reduced pressure and then drying the residue, or by adding a different organic solvent and then filtering the precipitated salt. At this time, the preferred salts may include salts derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid or toluenesulfonic acid, and the like.

The compound represented by Chemical Formula 1 or a pharmaceutically acceptable salt thereof has low water solubility, so an excessive amount of a solubilizing agent and an organic solvent are required in order to prepare into a formulation for injectional composition, such as an injectable pharmaceutical composition. However, an excessive amount of solubilizers and the like may cause hypersensitivity when administered to a patient. Therefore, in the present invention, a cyclodextrin and an isotonizing agent are used instead of using the solubilizer generally used in a formulation for injection, and the pH is adjusted, thereby obtaining a formulation for injection having both excellent solubility and stability of the compound represented by Chemical Formula 1.

Cyclodextrin, which is a component used in the formulation for injection according to the present disclosure is a cyclic oligosaccharide in which 6 to 12 glucose molecules are alpha-1,4-glycosidic bonds, and is used as a stabilizer in the present disclsoure. Preferably, the cyclodextrin is beta-cyclodextrin, or gamma-cyclodextrin, more preferably, beta-cyclodextrin. More preferably, the beta-cyclodextrin is (2-hydroxypropyl)-beta-cyclodextrin or sulfobutylether-beta-cyclodextrin, whose English abbreviations are 'HP-β-CD' and 'SBE-β-CD', respectively.

Among the stabilizers commonly used in a formulation for injection, the cyclodextrin is suitable for stabilizing the compound represented by Chemical Formula 1 or a pharmaceutically acceptable salt thereof.

Preferably, the cyclodextrin is used in an amount of 3.0 to 25.0 parts by weight with respect to 1 part by weight of the compound represented by Chemical Formula 1 or a pharmaceutically acceptable salt thereof. When the content is less than 3.0 parts by weight, it is not sufficient to stabilize the compound represented by Chemical Formula 1, which may cause a problem that rehydration of the formulation for injection is difficult or the total related substances increases during long-term storage. Further, when the content is greater than 25.0 parts by weight, the amount of the stabilizer used is too larger and thus, the viscosity of the formulation for injection become high, or there is a risk of causing hypersensitivity when administered to a patient.

More preferably, the content of the cyclodextrin is 3.5 parts by weight or more, 4.0 parts by weight or more, or 4.5 parts by weight or more; and 20.0 parts by weight or less, 19.0 parts by weight or less, 18.0 parts by weight or less, 17.0 parts by weight or less, 16.0 parts by weight or less, 15.0 parts by weight or less, 14.0 parts by weight or less, 13.0 parts by weight or less, 12.0 parts by weight or less, 11.0 parts by weight or less; or 10.0 parts by weight or less, with respect to 1 part by weight of the compound represented by Chemical Formula 1 or a pharmaceutically acceptable salt thereof.

Meanwhile, the 'isotonic agent' used in the formulation for injection according to the present disclosure is an additive added to make the osmotic pressure of the formulation for injection similar to the osmotic pressure in the body. Since the formulation for injection is administered directly into the body without a separate dilution process, it should be manufactured at the same osmotic pressure as the body in order to reduce side effects when administered in the body. Preferably, the isotonizing agent may be sodium chloride (NaCl), D-mannitol, dextrose, glycerin, or potassium chloride (KCI), more preferably, sodium chloride (NaCl), dextrose, glycerin, or potassium chloride (KCI), and most preferably, sodium chloride (NaCl), dextrose, or potassium chloride (KCI).

The isotonizing agent may differ in the content required to reach the osmolarity of the desired formulation for injection, depending on whether it is an electrolyte or a non-electrolyte. Therefore, the isotonizing agent is preferably contained so that the osmolality of the formulation for injection according to the present disclosure may be 100 to 700 mOsmol/L depending on the type of specific substance. More preferably, the osmolality of the formulation for injection may be 150 to 650 mOsmol/L, 150 to 450 mOsmol/L, 250 to 450 mOsmol/L, or 270 to 420 mOsmol/L.

Preferably, the pH of the formulation for injection according to the present invention is 5.0 to 6.0. Preferably, the formulation for injection of the present disclosure can have the above pH range due to the chemical properties of the liquid pharmaceutical composition itself of the present disclosure, and thus, the formulation for injection may not contain an additional pH adjusting agent for adjusting the pH. Here, the pH adjusting agent is a substance that adjusts the pH of the solution by adding the agent to thereby improve the solubility of poorly water-soluble or insoluble compounds, and a pharmaceutically acceptable acid or alkali agent is used. Examples thereof may include any one or more of hydrochloric acid, phosphoric acid, sodium hydroxide, potassium hydroxide, potassium monohydrogen phosphate, potassium dihydrogen phosphate, sodium monohydrogen phosphate, sodium dihydrogen phosphate, sodium carbonate, potassium carbonate and triethanolamine.

Preferably, the formulation for injection further includes a freeze-drying aid. Generally, the formulation for injection is mass-produced, then frozen, and stored and distributed under reduced pressure, which can enhance the stability of the active ingredient and improve the long-term storage stability. Therefore, the stability of the active substance must be maintained during the process of freeze-drying, and thus, in the present disclosure, a freeze-drying aid can be further included. Preferably, the freeze-drying aid is D-mannitol, sucrose, sorbitol, or trihalose, and more preferably, the freeze-drying aid is D-mannitol.

Preferably, the freeze-drying aid is used in an amount of 3.0 to 25.0 parts by weight with respect to 1 part by weight of the compound represented by Chemical Formula 1 or a pharmaceutically acceptable salt thereof. When the content is less than 3.0 parts by weight, it is not sufficient for stabilizing the compound represented by Chemical Formula 1, which may cause a problem that rehydration of the formulation for injection is difficult or the related substances increase during long-term storage. Further, when the content is greater than 25.0 parts by weight, the amount of the freeze-drying aid is too large, and thus, the viscosity of the formulation for injection become high, or there is a risk of causing hypersensitivity when administered to a patient.

More preferably, the content of the freeze-drying aid is 3.5 parts by weight or more, 4.0 parts by weight or more, or 4.5 parts by weight or more; and 20.0 parts by weight or less, 15.0 parts by weight or less, 13.0 parts by weight or less, 10.0 parts by weight or less, 9.0 parts by weight or less, 8.0 parts by weight or less, 7.0 parts by weight or less, or 6.0 parts by weight or less with respect to 1 part by weight of the compound represented by Chemical Formula 1 or a pharmaceutically acceptable salt thereof.

Preferably, the freeze-drying aid is used in an amount of 0.5 to 5.0 parts by weight with respect to 1 part by weight of the cyclodextrin. More preferably, the content of the freeze-drying aid is 0.6 parts by weight or more, 0.7 parts by weight or more, or 0.8 or more; and 4.5 parts by weight or less, 4.0 parts by weight or less, 3.5 parts by weight or less, 3.0 parts by weight or less, 2.5 parts by weight or less, 2.3 parts by weight or less, 2.0 parts by weight or less, 1.9 parts by weight or less, 1.8 parts by weight or less, 1.7 parts by weight or less; 1.6 parts by weight or less, 1.5 parts by weight or less, 1.4 parts by weight or less, 1.3 parts by weight or less, or 1.2 parts by weight or less with respect to 1 part by weight of the cyclodextrin.

Preferably, the formulation for injection may include a solvent commonly used in the art in order to prepare the pharmaceutical composition in liquid form. As an example, the solvent of the formulation for injection may be distilled water, water for injection, acetate buffer, or physiological saline.

Preferably, the compound represented by Chemical Formula 1 or a pharmaceutically acceptable salt thereof is contained in an amount of 1 to 8 mg/mL in the formulation for injection. That is, the content of the compound represented by Chemical Formula 1 or a pharmaceutically acceptable salt thereof can be defined as a value obtained by dividing the content (mg) of the compound represented by Chemical Formula 1 or a pharmaceutically acceptable salt thereof by the total volume (mL) of the formulation for injection.

More preferably, the compound represented by Chemical Formula 1 or a pharmaceutically acceptable salt thereof is contained in an amount of 2 mg/mL or more, 3 mg/mL or more, or 4 mg/mL or more, or 5 mg/mL or more; and 7 mg/mL or less, 6 mg/mL or less, or 5.5 mg/mL or less in the formulation for injection.

Further, if necessary, the formulation for injection according to the present disclosure may further include a preservative, an antioxidant, and the like. The preservative and the antioxidant are not particularly limited as long as they are commonly used in the technical field to which the present disclosure pertains.

In addition, the formulation for injection according to the present disclosure can be prepared by mixing the above-mentioned ingredients excluding the solvent with a solvent. In this process, the order of addition to the solvent of each component can be adjusted as needed, or all components can be mixed and added to the solvent before being added to the solvent. However, such a preparation method is not limited, and the preparation of the formulation for injection can be modified according to methods known in the art.

The prepared formulation for injection may be subjected to sterilization and/or filtration if necessary, and may be freeze-dried for storage and distribution.

### [Advantageous Effects]

As described above, the formulation for injection containing 1-(5-(2,4-difluorophenyl)-1-((3-fluorophenyl)sulfonyl)-4-methoxy-1H-pyrrol-3-yl)-N-methylmethanamine, or a pharmaceutically acceptable salt thereof according to the present disclosure satisfies a specific pH range and includes an isotonizing agent, whereby it has high solubility and can exhibit excellent stability, and thus, can be used as an formulation for injection useful for the prevention and treatment of gastrointestinal ulcers, gastritis, reflux esophagitis, or gastrointestinal damage caused by Helicobacter pylori.

### [DETAILED DESCRIPTION OF THE EMBODIMENTS]

Hereinafter, preferred examples are presented in order to help easy understanding of the present disclosure, but the following examples are for illustrative purposes only and the scope of the present disclosure is not limited thereby.

### Example 1

The solutions were respectively prepared by adjusting the pH with the composition shown in Table 1 using 40 mg of the hydrochloride salt of the compound represented by Chemical Formula 1 (hereinafter referred to as 'API').

After that, each prepared solution was filled in a vial and stored in liquid form in a harsh condition (60 °C, 80 % RH) chamber for 4 weeks, and then the stability was evaluated. The results are shown in Table 2 below. For the stability evaluation, the content of a related substance of a liquid solution was analyzed by HPLC, and the total amount of the detected related substance was measured.

**Table 1**

| | #1-1 | #1-2 | #1-3 | #1-4 | #1-5 |
|---|---|---|---|---|---|
| API | 40 mg | | | | |
| HP-β-CD | 200 mg | | | | |
| D-mannitol | 200 mg | | | | |
| Water for injection | 4 mL | | | | |
| pH (HCl / NaOH) | 3.0 | 4.0 | 5.0 | 6.0 | 7.0 |

**[Table 2]**

| | Total related substance % | |
|---|---|---|
| | Initial | 4-week |
| #1-1 | 0.21 | 1.48 |
| #1-2 | 0.18 | 0.44 |
| #1-3 | 0.17 | 0.30 |
| #1-4 | 0.15 | 0.25 |
| #1-5 | 0.17 | 0.69 |

As shown in Table 2, it was confirmed that when stored in a liquid solution state under harsh conditions for 4 weeks, the compositions of #1-2 to #1-4 having a pH of 4.0 to 6.0 have a stability that relatively, the production of the total related substance does not significantly increase.

### Example 2

In Example 1, the following experiment was performed by selecting pH 6.0 at which the production of total related substances is most low.

As shown in Table 3 below, the sample was prepared at different concentrations of API, the browning patterns of the properties depending on the concentration were confirmed. Each of the prepared solutions was visually evaluated, and the results are shown in Table 4 below.

**[Table 3]**

| | #1 -4 | #2 |
|---|---|---|
| API | 40 mg | 40 mg |
| HP-β-CD | 200 mg | 200 mg |
| D-mannitol | 200 mg | 200 mg |
| Water for injection | 4 mL | 10mL |
| pH | 6.0 | 6.0 |

**[Table 4]**

| | Properties | |
|---|---|---|
| | Initial | 4-week |
| #1-4 | colorless transparent liquid | browning |
| #2 | colorless transparent liquid | colorless transparent liquid |

As shown in Table 4 , it was confirmed that the properties of #2 having low concentration do not change even when stored under harsh conditions for 4 weeks.

### Example 3

The following experiment was performed by selecting the concentration (4 mg/mL) at which the properties were stable in Example 2.

As shown in Table 5 below, a sample solution was prepared by changing the amount of each isotonizing agent so that the osmotic pressure of the solution was 380 mOsmol/L (or similar to the osmotic pressure in the body). The prepared solution was stored for 4 weeks in a chamber under harsh conditions (60°C, 80% RH), and the properties and stability were evaluated by the same method as in Example 1. The results are shown in Table 6 below.

**[Table 5]**

| | | #2 | #3-1 | #3-2 | #3-3 | #3-4 |
|---|---|---|---|---|---|---|
| API | | 40 mg | | | | |
| HP-β-CD | | 200 mg | | | | |
| D-mannitol | | 200 mg | | | | |
| Water for injection | | 10 mL | | | | |
| pH | | 6.0 | | | | |
| Isotonizing agent | NaCl | - | 90 mg | - | - | - |
| | D-mannitol | - | - | 400 mg | - | - |
| | Dextrose | - | - | - | 400 mg | - |
| | KCl | - | - | - | - | 90 mg |

**[Table 6]**

| | Properties | | Total related substance % | |
|---|---|---|---|---|
| | Initial | 4-week | Initial | 4-week |
| #2 | colorless transparent liquid | colorless transparent liquid | 0.04 | 0.68 |
| #3-1 | colorless transparent liquid | colorless transparent liquid | 0.03 | 0.63 |
| #3-2 | colorless transparent liquid | colorless transparent liquid | 0.05 | 0.62 |
| #3-3 | colorless transparent liquid | colorless transparent liquid | 0.03 | 0.58 |
| #3-4 | colorless transparent liquid | colorless transparent liquid | 0.03 | 0.57 |

As shown in Table 6 above, it was confirmed that all the solutions to which isotonizing agents were added do not change in the properties even after being stored under harsh conditions for 4 weeks. And, particularly, it was confirmed that the compositions of #3-1 to #3-4 show a more stable level of the production of the total related substance than a comparative group #2.

### Reference Example 1

Among the isotonizing agents that were shown to be relatively stable in Example 3, NaCl was selected and the following experiment was performed.

As shown in Table 7 below, each solution was prepared without adding a pH adjuster or by changing the type. The prepared solution was stored for 4 weeks in a chamber under harsh conditions (60°C, 80% RH), and the properties and stability were evaluated by the same method as in Example 1. The results are shown in Table 6 below.

**Table 7**

| | | #4-1 | #3-1 | #4-2 |
|---|---|---|---|---|
| API | | 40 mg | | |
| HP-β-CD | | 200 mg | | |
| D-mannitol | | 200 mg | | |
| NaCl | | 90 mg | | |
| Water for injection | | 10 mL | | |
| pH adjusting agent | HCl / NaOH | - | proper amount | - |
| | Phosporic acid / sodium dihydrogen phosphate | - | - | proper amount |

**[Table 8]**

| | Properties | | Total related substance % | |
|---|---|---|---|---|
| | Initial | 4-week | Initial | 4-week |
| #4-1 | colorless transparent liquid | colorless transparent liquid | 0.05 | 0.51 |
| #3-1 | colorless transparent liquid | colorless transparent liquid | 0.03 | 0.63 |
| #4-2 | colorless transparent liquid | browning | 0.03 | 0.84 |

As shown in Table 8, it was confirmed that #4-1 not containing a pH adjuster has excellent stability because the total amount of related substances is less than that of #3-1 or #4-2 containing a pH adjuster.

### Reference Example 2

Two experimental groups (with/without HCl/NaOH pH adjuster) confirmed in Example 3 and Reference Example 1 were selected, and a long-term storage experiment in the liquid composition state was performed. The composition of each experimental group is shown in Table 9 below.

Each prepared solution was stored in a chamber under an accelerated condition (40°C, 75% RH) for 6 months, and the properties and stability were evaluated by the same method as in Example 1. The results are shown in Table 10 below.

**[Table 9]**

| | #4-1 | #3-1 |
|---|---|---|
| API | 40 mg | |
| HP-β-CD | 200 mg | |
| D-mannitol | 200 mg | |
| NaCl | 90 mg | |
| Water for injection | 10 mL | |
| HCl / NaOH | Not added | Added |

**[Table 10]**

| | Properties | | | Total related substance % | | |
|---|---|---|---|---|---|---|
| | Initial | Accelerated 3-month | Accelerated 6-month | Initial | Accelerated 3-month | Accelerated 6-month |
| #4-1 | colorless transparent liquid | colorless transparent liquid | colorless transparent liquid | 0.05 | 0.16 | 0.18 |
| #3-1 | colorless transparent liquid | colorless transparent liquid | colorless transparent liquid | 0.05 | 0.22 | 0.30 |

As shown in Table 10, it was confirmed that although #4-1 containing no pH adjuster was stored in a liquid state for a long period of time, there is no change in the properties and there is almost no productin of the total related substance, thereby having excellent stability.

## Claims

1. A formulation for injection comprising a compound represented by the following Chemical Formula 1 or a pharmaceutically acceptable salt thereof; a cyclodextrin; and an isotonizing agent,
wherein the formulation for injection has a pH of 4.0 to 6.0:

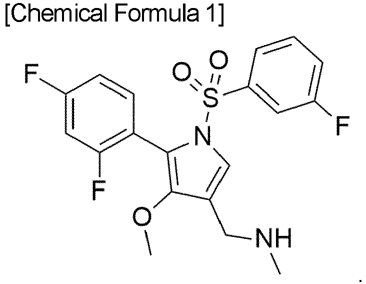

2. The formulation for injection of claim 1, wherein
the cyclodextrin is (2-hydroxypropyl)-beta-cyclodextrin, or sulfobutylether-beta-cyclodextrin.

3. The formulation for injection of claim 1, wherein
the cyclodextrin is contained in an amount of 4.5 to 15.0 parts by weight with respsect to 1 part by weight of the compound represented by Chemical Formula 1 or a pharmaceutically acceptable salt thereof.

4. The formulation for injection of claim 1, wherein
the isotonizing agent is sodium chloride (NaCl), D-mannitol, dextrose, glycerin or potassium chloride (KCI).

5. The formulation for injection of claim 1, wherein
the formulation for injection has an osmolarity of 100 to 700 mOsmol / L.

6. The formulation for injection of claim 1, wherein
the pH is 5.0 to 6.0.

7. The formulation for injection of claim 1, wherein
the formulation for injection does not comprise a pH adjusting agent.

8. The formulation for injection of claim 1, wherein
the formulation for injection further comprises a freeze-drying aid, and
the freeze-drying aid is D-mannitol, sucrose, sorbitol, or trihalose.

9. The formulation for injection of claim 8, wherein
the freeze-drying aid is contained in an amount of 3.0 to 25.0 parts by weight with respect to 1 part by weight of the compound represented by Chemical Formula 1 or a pharmaceutically acceptable salt thereof.

10. The formulation for injection of claim 1, wherein
a solvent of the formulation for injection is distilled water, water for injection, acetate buffer, or physiological saline.

11. The formulation for injection of claim 1, wherein
the compound represented by Chemical Formula 1 or a pharmaceutically acceptable salt thereof is contained in an amount of 1 to 8 mg/mL in the formulation for injection.
